# EUROPÄISCHE PATENTSCHRIFT

(11) **EP 3 120 765 B1**
(45) Veröffentlichungstag und Bekanntmachung des Hinweises auf die Patenterteilung: **16.05.2018**
(21) Anmeldenummer: 16178633.0
(22) Anmeldetag: 08.07.2016
(51) Int. Cl.: A61B 5/05, G01R 33/12, A61B 5/00

(54) **MPI-VERFAHREN**
MPI METHOD
PROCEDE MPI

(30) Priorität: 24.07.2015 DE 102015214071
(43) Veröffentlichungstag der Anmeldung: 25.01.2017
(73) Patentinhaber: Bruker BioSpin MRI GmbH, 76275 Ettlingen (DE)
(72) Erfinder: Weber, Alexander, 76185 Karlsruhe (DE)
(74) Vertreter: Kohler Schmid Möbus Patentanwälte

(56) Entgegenhaltungen:
- DE-A1-102012 211 662

## Beschreibung

### Hintergrund der Erfindung

Die Erfindung betrifft ein MPI-Verfahren zur Lokalisierung von Magnetpartikeln innerhalb einer Messprobe, wobei ein ortsabhängiges Magnetfeld angelegt wird, welches eine feldfreie Region aufweist, umfassend folgende Verfahrensschritte:
- Festlegen eines Kalibrationsvolumens und eines Messvolumens, wobei das Kalibrationsvolumen größer ist als das Messvolumen und wobei das Messvolumen vollständig innerhalb des Kalibrationsvolumens angeordnet ist;
- Erfassen von Kalibrationssignalen Sⱼ und Erstellung einer Systemmatrix S aus den Kalibrationssignalen Sⱼ
- Aufnahme eines MPI-Messsignals u (MPI-Zeitsignals oder MPI-Frequenzspektrum, das durch Fouriertransformation aus dem MPI-Zeitsignal erhalten wird), wobei durch Anlegen des magnetischen Ansteuerungsfeldes die feldfreie Region mit Hilfe einer Messsequenz durch das Messvolumen bewegt wird;
- Rekonstruktion einer ortsabhängigen Magnetpartikelkonzentration mit Magnetpartikelkonzentrationswerten cᵢ innerhalb des Kalibrationsvolumens aus dem aufgenommenen MPI-Messsignal u und der erstellten Systemmatrix S und Zuordnung der Magnetpartikelkonzentrationswerte cᵢ zu Voxeln im Kalibrationsvolumen.

Ein solches Verfahren ist beispielsweise bekannt aus [01] - [03].

Magnetic-Particle-Imaging (abgekürzt "MPI") ist ein bildgebendes Verfahren, das es ermöglicht, die örtliche Verteilung superparamagnetischen Nanopartikel (hier als Magnetpartikel bezeichnet) zu bestimmen. Hierzu werden die Magnetpartikel in einem Messvolumen verschiedenen statischen und dynamischen Magnetfeldern ausgesetzt und die Magnetisierungsänderungen der Magnetpartikel mittels Empfangsspulen detektiert. Zur Ortskodierung wird bei MPI ein magnetisches Gradientenfeld im Bereich des Messvolumens angelegt, welches eine feldfreie Region aufweist. Mittels eines dynamischen Magnetfeldes (Drive-Feld) und/oder homogenen Fokusfeldern wird die feldfreie Region entlang einer vordefinierten Trajektorie (vorgegebener Verlauf jedes Punktes der feldfreien Region) innerhalb des Messvolumens verschoben. Durch das Überfahren der Magnetpartikel mit der feldfreien Region und der damit verbundenen Magnetisierungsänderung wird ein Messsignal erzeugt, das mit Empfangsspulen detektiert wird.

Zur Kalibrierung des Systems wird eine Systemmatrix erfasst. Dazu kann bspw. eine Kalibrationsmessung durchgeführt werden, bei der für jeden Messpunkt ein Kalibrationssignal aufgenommen und in der Systemmatrix hinterlegt wird. Die Kalibrationsmessung wird auch für Positionen der Kalibrierprobe außerhalb des Messvolumens durchgeführt, so dass ein MPI-Bild eines Volumens rekonstruiert werden kann, welches insgesamt größer ist als das Messvolumen. Während in [01] und [02] MPI-Messungen an einem Phantom mit Magnetpartikel durchgeführt werden, bei dem sich sämtliche Magnetpartikel innerhalb des Messvolumens befinden, wird in [03] ein Verfahren zur in vivo Aufnahme eines Rattenherzes beschrieben. Bei in-vivo Aufnahmen befinden sich Magnetpartikel auch außerhalb des Messvolumens. Diese werden zwar nicht von der feldfreien Region überstrichen, tragen aber auf Grund ihrer Rotation und der Unschärfe der feldfreien Region ebenfalls zum Messsignal bei, was zu Artefakten im MPI-Bild führen kann. Ein weiteres MPI Verfahren ist aus der DE 10 2012 211662 bekannt.

### Aufgabe der Erfindung

Es ist Aufgabe der vorliegenden Erfindung, ein Verfahren insbesondere für in-vivo MPI-Aufnahmen vorzuschlagen, mit dem, auch bei hohen Magnetpartikeldichten außerhalb des Messvolumens, zeitsparend artefaktarme Bilddaten erzeugt werden können.

### Kurze Beschreibung der Erfindung

Diese Aufgabe wird erfindungsgemäß durch ein Verfahren gemäß Patentanspruch 1 gelöst.
Sowohl das Messvolumen als auch das Kalibrationsvolumen umfasst eine Vielzahl an Voxeln (Volumeneinheiten), wobei die Größe eines Voxels durch die Abstände der Kalibrierprobepositionen festgelegt wird, die für die Kalibration des Messvolumens verwendet wird. Für eine bevorzugte Anwendung entspricht die Größe eines Voxels der Größe der Kalibrierprobe und die Kalibrierprobenposition entspricht dem Zentrum des Voxels.
Erfindungsgemäß werden diejenigen Magnetpartikelkonzentrationswerte, welche Voxeln außerhalb des Messvolumens zugeordnet wurden, verworfen, und es wird ein MPI-Bild erstellt, welches ausschließlich Magnetpartikelkonzentrationswerte enthält, die den Voxeln eines Voxelgitters innerhalb des Messvolumens zugeordnet wurden.

Als Kalibrationssignale können Zeitsignale erfasst werden oder Frequenzspektren, die durch Fouriertransformation aus Zeitsignalen erhalten werden.

Um den Einfluss der außerhalb des Messvolumens lokalisierten Magnetpartikel auf das Messergebnis zu minimieren, werden auch Kalibrationssignale für Probenpositionen außerhalb des Messvolumens erfasst ("overscanning"). Im Gegensatz zum Stand der Technik werden jedoch diejenigen rekonstruierten Magnetpartikelkonzentrationswerte, welche Voxeln außerhalb des Messvolumens zugeordnet wurden, verworfen und es wird ein MPI-Bild erstellt, welches ausschließlich Magnetpartikelkonzentrationswerte enthält, die den Voxeln innerhalb des Messvolumens zugeordnet wurden.

Im Rahmen der Erfindung wurde erkannt, dass bei der Rekonstruktion über ein Kalibrationsvolumen, welches größer als das Messvolumen gewählt wurde, Partikelsignale von Magnetpartikeln außerhalb des Messvolumens unabhängig von ihrer tatsächlichen Position an die äußere Grenze des Messvolumens projiziert werden, so dass es nach der Rekonstruktion zu einer Fehlinterpretation des Overscan-Bereichs (Bereich des Kalibrationsvolumens außerhalb des Messvolumens) kommen kann. Durch das erfindungsgemäße "overscanning" bei der Erfassung der Kalibrationssignale (Kalibrationsvolumen > Messvolumen) und die Rekonstruktion des gesamten Kalibrationsvolumens in Kombination mit dem Verwerfen der Magnetpartikelkonzentrationswerte für Voxel außerhalb des Messvolumens, wird sichergestellt, dass einerseits der Einfluss der außerhalb des Messvolumens angeordneten Magnetpartikel auf MPI-Signale innerhalb des Messvolumens berücksichtigt wird und andererseits der Einfluss der außerhalb des Messvolumens angeordneten Magnetpartikel auf rekonstruierte Daten außerhalb des Messvolumens nicht in das MPI-Bild, welches zur weiteren Auswertung/Interpretation verwendet wird, eingehen, und somit das MPI-Bild lediglich vertrauenswürdige MPI-Daten (Magnetpartikelkonzentrationswerte) enthält.

Das Verwerfen der Magnetpartikelkonzentrationswerte außerhalb des Messvolumens kann vor oder nach Erstellung einer Bilddatei erfolgen. Entscheidend ist, dass die den Voxeln außerhalb des Messvolumens zugeordneten Magnetpartikelkonzentrationswerte in dem für eine weitere Auswertung verwendeten MPI-Bild nicht erfasst werden, um eine Fehlinterpretation der im MPI-Bild enthaltenen Daten zu vermeiden.

Das Messvolumen wird benutzerdefiniert ausgewählt und wird je nach Spulentopografie definiert durch Anregungsfeld und/oder Gradientenfelder und/oder Fokusfelder.

Die feldfreie Region kann bspw. ein feldfreier Punkt oder eine feldfreie Linie sein, der/die durch die Messsequenz entlang einer Trajektorie durch das Messvolumen bewegt wird.

Das erfindungsgemäße Verfahren ist insbesondere vorteilhaft, wenn Partikelkonzentrationen von Bereichen ermittelt werden sollen, die größer als das Messvolumen sind. Dazu müssen mehrere MPI-Messungen durchgeführt werden, wobei das Messvolumen entsprechend verschoben werden muss und die Gesamtheit der Messvolumina den zu vermessenden Bereich bilden. Gemäß der Erfindung dürfen die rekonstruierten Magnetpartikelkonzentrationswerte außerhalb des Messvolumens nicht in die letztlich ermittelte und als MPI-Bild dargestellte ortsaufgelöste Magnetpartikelkonzentration eingehen. Für die Aufnahme von mehreren aneinander angrenzenden Messvolumina müssen erfindungsgemäß die Kalibrationsvolumina so gewählt werden, dass sie einander (um den Overscan-Bereich) überlappen.

### Vorteilhafte Ausführungsform der Erfindung

Vorzugsweise umgibt das Kalibrationsvolumen das Messvolumen, d.h. das Kalibrationsvolumen überragt das Messvolumen in jeder Richtung. Auf diese Weise werden Magnetpartikel, die sich auf verschiedene Seiten außerhalb des Messvolumens befinden, gleichermaßen berücksichtigt.

Eine besonders bevorzugte Variante des erfindungsgemäßen Verfahrens sieht vor, dass das Kalibrationsvolumen das Messvolumen in jeder Richtung um jeweils ein einziges Voxel überragt. Die benötigte Zeit für die Kalibration und die Rekonstruktion kann dadurch klein gehalten werden. Insbesondere bei einem kleinem Verhältnis der Magnetpartikelkonzentration außerhalb des Messvolumens zu der Magnetpartikelkonzentration innerhalb des Messvolumens wird aufgrund des bei der Messung auftretenden Rauschens im Falle eines größer gewählten Overscans keine wesentliche Verbesserung erreicht, so dass ein Overscan mit einer Dicke von einem einzigen Voxel ausreichend ist.

Bei einer bevorzugten Variante des erfindungsgemäßen Verfahrens werden im Bereich außerhalb des Messvolumens die Kalibrationssignale mit einer kleineren Auflösung erfasst als innerhalb des Messvolumens. Der Overscan-Bereich kann somit zeitsparend gescannt werden.

Dies kann beispielsweise erfolgen, indem in einem Bereich außerhalb des Messvolumens weniger Kalibrationssignale aufgenommen werden als Voxelpositionen in diesem Bereich vorhanden sind. Außerhalb des Messvolumens werden also nicht für alle Voxel Kalibrationssignale erfasst. Dabei kann das Voxelgitter des Messvolumens auf das gesamte Kalibrationsvolumen erweitert sein (gleiches Voxelgitter für Messvolumen und Overscan-Bereich).

Alternativ oder zusätzlich kann vorgesehen sein, dass für die Erfassung der Kalibrationssignale außerhalb des Messvolumens eine größere Kalibrierprobe als für die die Erfassung der Kalibrationssignale innerhalb des Messvolumens verwendet wird.

Darüber hinaus kann vorgesehen sein, dass für die Erfassung der Kalibrationssignale außerhalb des Messvolumens ein anderes Voxelgitter, insbesondere ein Voxelgitter mit größeren Voxeln, als für die die Erfassung der Kalibrationssignale innerhalb des Messvolumens verwendet wird. Eine Veränderung des Voxelgitters im Overscan-Bereich bedeutet, dass die Kalibrierprobe an andere Kalibrierprobenpositionen verschoben wird. Das Voxelgitter wird in diesen Fall also an die gewünschte Auflösung im Overscan-Bereich angepasst.

Es ist auch eine denkbar, für die Erfassung der Kalibrationssignale außerhalb des Messvolumens alle drei Maßnahmen vorzusehen, also eine größere Kalibrierprobe zu verwenden, das Voxelgitter anzupassen und weniger Kalibrationssignale aufzunehmen als Voxelpositionen in diesem Bereich vorhanden sind.

Vorzugsweise wird das Verfahren zum Vermessen von solchen Messproben eingesetzt, welche zumindest einen zum Messvolumen benachbarten Bereich aufweisen, in dem eine Magnetpartikelkonzentration herrscht, die größer Null (d.h. es befinden sich Magnetpartikel außerhalb des Messvolumens, beispielsweise bei in-vivo-Messungen), vorzugsweise größer oder gleich der Magnetpartikelkonzentration im Messvolumen ist. Insbesondere im letzteren Fall ist der Einfluss der Magnetpartikel sowohl auf Bereiche innerhalb des Messvolumens als auch auf Bereiche des Kalibrationsvolumens außerhalb des Messvolumens groß. Mit herkömmlichen Verfahren können daher erhebliche Artefakte entstehen.

Vorzugsweise wird zur Erfassung der Kalibrationssignale eine Kalibrationsmessung durchgeführt, wobei für verschiedenen Positionierungen der Kalibrierprobe innerhalb des Kalibrationsvolumens jeweils ein Kalibrationssignal Sⱼ detektiert wird, wobei die feldfreie Region während der Detektion jedes Kalibrationssignals Sⱼ durch Anlegen des magnetischen Ansteuerungsfeldes gemäß der Messsequenz durch das Messvolumen bewegt wird.

Für die Kalibration der Systemmatrix wird eine Kalibrierprobe an verschiedene Voxelpositionen bewegt und jeweils die Systemantwort (Kalibrationssignal Sⱼ) gemessen. Auf Grund von Temperatureffekten kann es zu Beginn der Messung zu Drifts auf den einzelnen Frequenzen der Kalibrationssignale kommen. Da gemäß der Erfindung die rekonstruierten Werte für Voxel außerhalb des Messvolumens nach der Rekonstruktion wieder verworfen werden, ist es vorteilhaft, wenn bei der Kalibrationsmessung zuerst die Kalibrationssignale für Probenpositionen außerhalb des Messvolumens und danach für Probenpositionen innerhalb des Messvolumens gemessen werden. Die Drifteffekte wirken sich dann nicht wesentlich auf das MPI-Bild aus.

Vorzugsweise wird das erfindungsgemäße Verfahren für in-vivo-Aufnahmen verwendet. Die Vorteile der Erfindung kommen hier besonders gut zur Geltung, da bei in-vivo Aufnahmen in der Regel eine nicht zu vernachlässigende Magnetpartikelkonzentration außerhalb des Messvolumens vorhanden ist.

### Zeichnung und detaillierte Beschreibung eines Ausführungsbeispiels

- Fig. 1: zeigt ein Verlaufsdiagram des erfindungsgemäßen Verfahrens.
- Fig. 2: zeigt ein Messvolumen mit einer Lissajous-Trajektorie (2D-Darstellung).
- Fig. 3: zeigt das Messvolumen Fig. 2 und ein Kalibrationsvolumen mit einem Overscan von einer einzigen Voxelreihe (2D-Darstellung).
- Fig. 4: zeigt das Messvolumen aus Fig. 2 und ein Kalibrationsvolumen mit einem Overscan von zwei Voxelreihen (2D-Darstellung).
- Fig. 5: zeigt das Messvolumen aus Fig. 2 und ein Kalibrationsvolumen mit einem Overscan von zwei Voxelreihen (bzgl. dem Voxelgitter des Overscan-Bereichs), wobei außerhalb des Messvolumens ein Voxelgitter mit halber Auflösung verwendet wird. Die Größe der Kalibrierprobe wird an die Voxelgröße angepasst, sodass für den Overscan-Bereich eine Kalibrierprobe verwendet wird, die viermal größer ist als im Messvolumen. (2D-Darstellung).
- Fig. 6: zeigt ein Referenzbild einer Magnetpartikelverteilung für eine Simulation.
- Fig. 7: zeigt Simulationsdaten der Magnetpartikelverteilung aus Fig. 6 ohne Overscan.
- Fig. 8: zeigt Simulationsdaten der Magnetpartikelverteilung aus Fig. 6 mit einem Overscan von zwei Voxelreihen.
- Fig. 9: zeigt Simulationsdaten der Magnetpartikelverteilung aus Fig. 6 mit einem Overscan von zwei Voxelreihen (bzgl. dem Voxelgitter des Overscan-Bereichs) wobei im Overscan-Bereich ein Voxelgitter mit halber Auflösung verwendet wird. Die Größe der Kalibrierprobe wird an die Voxelgröße angepasst, sodass für den Overscan-Bereich eine Kalibrierprobe verwendet wird, die viermal größer ist als im Messvolumen.
- Fig. 10: zeigt das Ergebnis des erfindungsgemäßen Verfahrens mit einem Overscan gemäß Fig. 9.

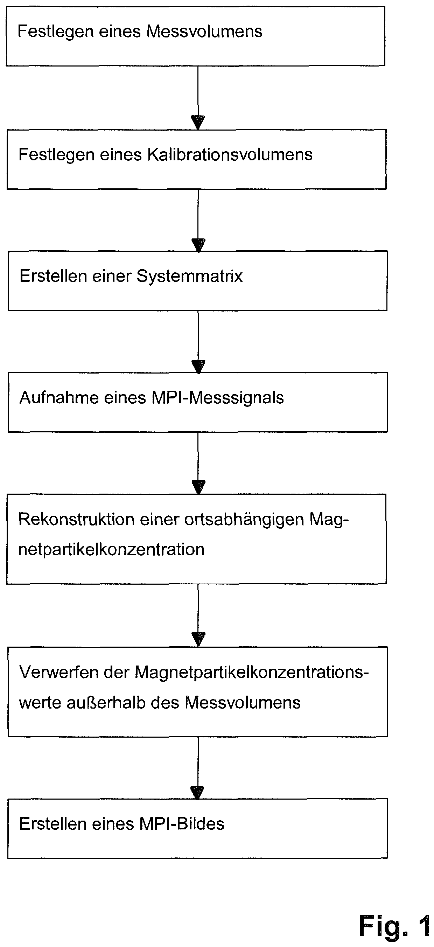

Die in **Fig. 1** aufgeführten Verfahrensschritte des erfindungsgemäßen Verfahrens werden im Folgenden beschrieben:
Zunächst wird ein benutzerdefiniertes Messvolumen sowie ein Voxelgitter für das Messvolumen ausgewählt, in welchem bei der späteren MPI-Messung eine feldfreie Region über die einzelnen Voxel des Voxelgitters des Messvolumens bewegt wird. Dazu werden entsprechende Magnetfelder (Gradientenfelder, Anregungsfelder, Fokusfelder) angelegt, wobei die Größe des Messvolumens von der Amplitude dieser Magnetfelder abhängt, bzw. die Magnetfelder so eingestellt werden, dass sich die gewünschte Größe des Messvolumens ergibt.

Zur Erstellung einer Systemmatrix wird innerhalb eines zuvor gewählten Kalibrationsvolumens, welches größer ist als das Messvolumen (Kalibrationsvolumen = Messvolumen + Overscan-Bereich) und dieses beinhaltet, eine Kalibrierung vorgenommen. Für den Overcan-Bereich kann dabei ein vom Voxelgitter des Messvolumens verschiedenes Voxelgitter gewählt werden oder das Voxelgitter des Messvolumens kann auf das gesamte Kalibrationsvolumen erweitert werden. Die Kalibrierung kann durch Simulation erfolgen oder durch eine Kalibrationsmessung. Für eine Kalibrationsmessung wird eine kleine mit Magnetpartikeln gefüllte Probe (Kalibrierprobe) verwendet, wobei insbesondere für die Erfassung der Kalibrationssignale innerhalb und außerhalb des Messvolumens verschiedene Kalibrierproben verwendet werden können. Die Kalibrierprobe wird z.B. mit Hilfe eines Positionierungsroboters an die verschiedenen Positionen innerhalb des Kalibrationsvolumens bewegt. Statt die Kalibrierprobe zu bewegen, können mit Hilfe von zusätzlichen Magnetfeldern die magnetischen Verhältnisse von verschiedenen Probenorten nachgebildet werden. Dadurch verkürzt sich die Kalibrationszeit, da kein Roboter bewegt werden muss. Für jede Kalibrierprobenposition im Kalibrationsvolumen wird unter denselben Bedingungen (gleiche Messsequenz) wie bei der eigentlichen MPI-Messung (Aufnahme der Messsignale) ein Kalibrationssignal aufgenommen. Es werden also für jeden Messpunkt innerhalb des Messvolumens und für zusätzliche Messpunkte außerhalb des Messvolumens ortsabhängig Kalibrationssignale Sⱼ aufgenommen. Aus den Kalibrationssignalen wird dann die Systemmatrix S erstellt, indem die einzelnen Kalibrationssignale Sⱼ, also die einzelnen Frequenzspektren bzw. die einzelnen Zeitsignale, spaltenweise eingetragen werden.

Anschließend wird die eigentliche MPI-Messung durchgeführt, indem ein MPI-Signal (Messvektor u) aufgenommen wird während die feldfreie Region gemäß der Messsequenz das Messvolumen durchläuft.

Im Rahmen einer Rekonstruktion wird dann mit Hilfe der ermittelten Systemmatrix S und dem Messvektor u ein Magnetpartikelkonzentrationsvektor c durch Auflösung des linearen Gleichungssystems u = S•c bestimmt. Es wird also eine ortsabhängigen Konzentrationsverteilung mit Magnetpartikelkonzentrationswerten cᵢ (Magnetpartikelverteilung) für das gesamte Kalibrationsvolumen rekonstruiert, wobei die Ortsabhängigkeit der Magnetpartikelkonzentrationswerte cᵢ aus der Systemmatrix S abgeleitet wird.

Gemäß der Erfindung werden diejenigen Magnetpartikelkonzentrationswerte cᵢ, die Bereichen (Voxeln) außerhalb des Messvolumens zugeordnet wurden, verworfen.

Erst dann wird das MPI-Bild erstellt, und zwar ausschließlich für Magnetpartikelkonzentrationswerte cᵢ, die Voxeln innerhalb des Messvolumens zugeordnet wurden. Das als Ergebnis ermittelte MPI-Bild enthält also ausschließlich Partikelkonzentrationswerte cᵢ für Voxel innerhalb des Messvolumens.

Gemäß der Erfindung wurde erkannt, dass die im Stand der Technik im MPI-Bild enthaltenen Magnetpartikelkonzentrationswerte außerhalb des Messvolumens Artefakte enthalten, insbesondere bei Aufnahmen eines Messvolumens, welches sich benachbart zu einem Bereich befindet, welche eine hohe Magnetpartikeldichte aufweist. Durch das erfindungsgemäße Verfahren wird einerseits gewährleistet, dass der Einfluss von Magnetpartikeln außerhalb des Messvolumens auf die Messsignale innerhalb des Messvolumens berücksichtigt wird; andererseits wird sichergestellt, dass die für Voxel außerhalb des Messvolumens ermittelten Magnetpartikelkonzentrationswerte nicht für weitere Berechnungen oder Messungen verwendet werden, da diese nicht vertrauenswürdig sind.

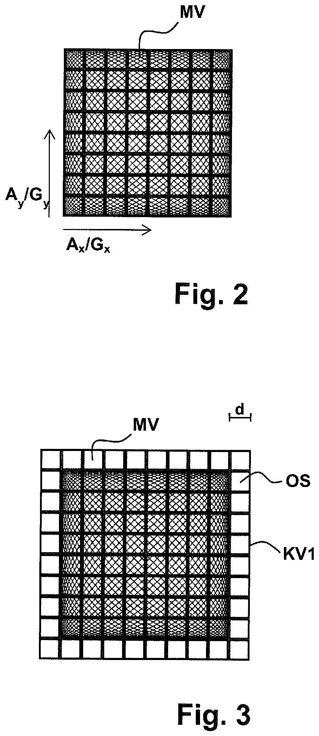

**Fig. 2** zeigt ein Messvolumen **MV** mit einer Lissajous-Trajektorie, entlang der ein feldfreier Punkt mittels eines Drive-Feldes (Überlagerung eines sinusförmigen Anregungsfeldes **A** und eines Gradientenfeldes **G**) durch das Messvolumen MV bewegt wird. Das Messvolumen MV ist in Voxel aufgeteilt.

In **Fig. 3-5** ist das Messvolumen MV aus Fig. 2 mit verschiedenen Kalibrationsvolumina **KV1, KV2, KV3** dargestellt, welche das Messvolumen MV an allen Seiten überlappt und somit eine Umhüllende bildet. Der Überlapp des Kalibrationsvolumens (Overscan-Bereich **OS1, OS2, OS3)** über das Messvolumen MV hinaus bildet also eine Umhüllende des Messvolumens MV.

**Fig. 3** zeigt eine besonders bevorzugte Wahl des Kalibrationsvolumens KV1. In diesem Fall wird im Kalibrierschritt neben dem Messvolumen V eine einzige zusätzliche Voxelreihe (2D-Messung), die über das Messvolumen MV hinausragt, aufgenommen (Overscan-Bereich OS1). Im Falle einer 3D-Messung umfasst der Overscan-Bereich OS1 eine Umhüllende des Messvolumens MV mit einer Dicke **d** von einem einzigen Voxel. Experimentelle Messungen haben gezeigt, dass dies in vielen Fällen ausreichend ist, um bei der Bildrekonstruktion die Gesamtheit der Magnetpartikel jenseits des Messvolumens MV im Systemmatrixverfahren herauszurechnen.

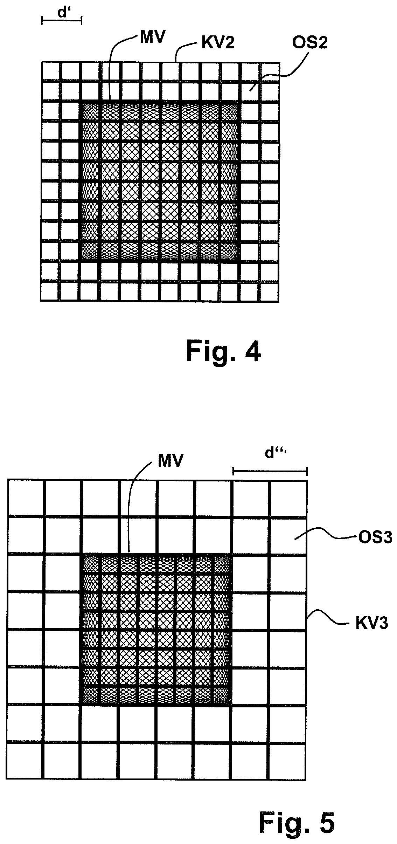

**Fig. 4** zeigt eine alternative Variante der Wahl des Kalibrationsvolumens KV2. Das Kalibrationsvolumen KV2 überragt das Messvolumen MV in diesem Fall um zwei Voxelreihen, wobei die Voxelgröße definiert wird durch die Abstände der Kalibrierprobepositionen im Messvolumen MV. Der Overscan-Bereich OS2 weist demnach eine Dicke **d'** von zwei Voxeln auf.

Überraschenderweise wurde festgestellt, dass es nicht erforderlich ist, den kompletten Bereich außerhalb des Messvolumens, in dem sich Magnetpartikel befinden, im Rahmen der Kalibrationsmessung zu vermessen, um ein zuverlässiges Ergebnis zu erhalten. Es wurde nämlich erkannt, dass sämtliche Magnetpartikel im Bereich außerhalb des Messvolumens MV ein ähnliches Bild an den Übergang vom Messvolumen MV zum Overscan-Bereich OS1, OS2, OS3 projizieren. Der Overscan-Bereich OS1, OS2, OS3 kann also mit einer geringeren Auflösung abgetastet werden als das Messvolumen MV, ohne wesentliche Informationen zu verlieren. In **Fig. 5** ist beispielsweise ein Overscan-Bereich OS3 gezeigt, der zwei Voxelreihen (bezogen auf das Voxelgitter des Overscan-Bereichs OS3) umfasst, wobei hier für den Overscan-Bereich ein anderes Voxelgitter verwendet wurde als für das Messvolumen. Ein Voxel des Voxelgitters des Overscan-Bereichs (OS-Voxel - in Fig. 5 als große Quadrate im Overscan-Bereich OS3 dargestellt) entspricht vier Voxeln des Voxelgitters des Messvolumens) (d"= 4 Voxel des Voxelgitters des Messvolumens). Für jedes OS-Voxel wird lediglich ein einziges Kalibrationssignal aufgenommen, d.h. der Overscan-Bereich OS3 wird in diesem Fall entweder mit einer Kalibrierprobe vermessen, die größer ist (hier: 2x2 Voxel) als die Kalibierprobe, mit der das Messvolumen MV vermessen wird (1 Voxel), und/oder die Kalibrierprobe wird innerhalb des Abschnitts der 2x2 Voxel an einer beliebigen Position (z.B. an einer der vier Voxelpositionen des OS-Voxels oder im Zentrum des OS-Voxels) vermessen.

Im Folgenden werden Simulationsdaten zu verschiedenen Overscan-Bereichen gezeigt: Es wurden MPI-Daten für eine 2D-Messequenz simuliert. Die Voxelgröße entspricht 1 x 1 mm² und das Messvolumen beträgt 32 x 32 mm².

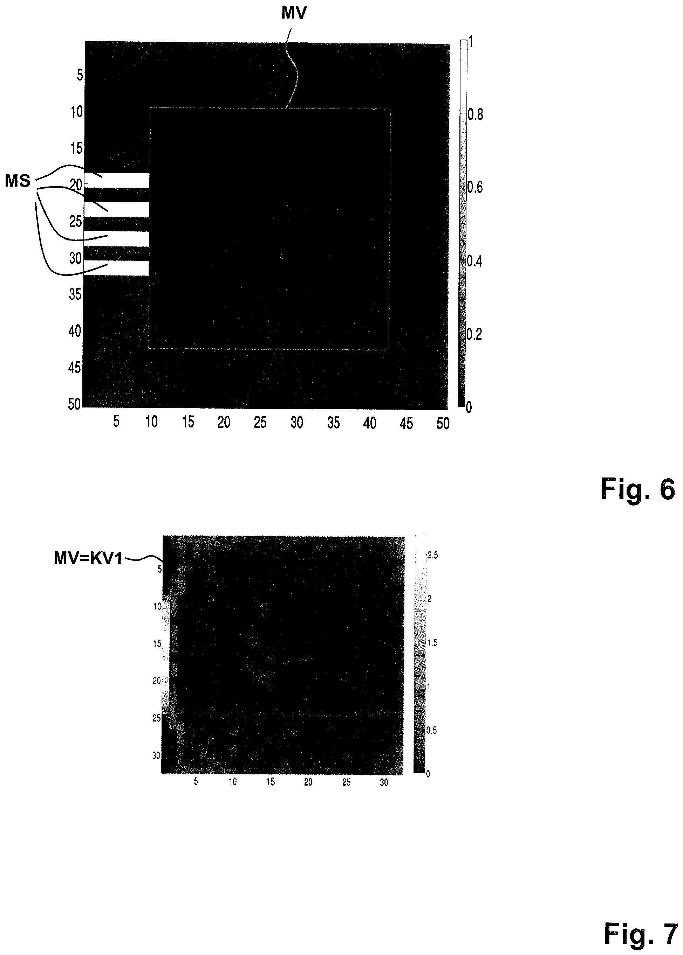

**Fig. 6** zeigt ein Referenzbild einer Simulation des erfindungsgemäßen Verfahrens für den Extremfall einer Magnetpartikeldichte von Null innerhalb des Messvolumens MV. Außerhalb des Messvolumens MV sind vier Magnetpartikel-Streifen **MS** zu jeweils 2x9 Voxeln mit der Intensität 1 angrenzend an die linke Seite des Messvolumens angeordnet. Eine Intensität von 1 bedeutet hierbei, dass sich in diesem Voxel während der Objektmessung die gleiche Menge an Magnetpartikeln wie während der Kalibrationsmessung in der Kalibrierprobe befunden haben (relative Intensität bezogen auf Kalibrationsmessung). Die Magnetpartikeldichte ist hier also außerhalb des Messvolumens MV deutlich größer als innerhalb des Messvolumens MV.

**Fig. 7-9** zeigen simulierte Bilddaten, wobei der Overscan-Bereich **OS2, OS3** variiert wurde. In allen Fällen war der Overscan-Bereich OS2, OS3 kleiner als der Bereich, innerhalb dem die Gesamtheit aller Magnetpartikel angeordnet war. Die in den Fig. 7-9 gezeigten Bilddaten decken jeweils das gesamten Kalibrationsvolumen **KV1, KV2, KV3** ab, beinhalten also sämtliche aus der Rekonstruktion gewonnenen Daten.

**Fig. 7** zeigt simulierte rekonstruierte Daten ohne Overscan (Kalibrationsvolumen KV = Messvolumen MV). Man kann deutlich erkennen, dass am linken Rand innerhalb des Messvolumens Artefakte mit hoher Intensität (> 2.5) auftreten.

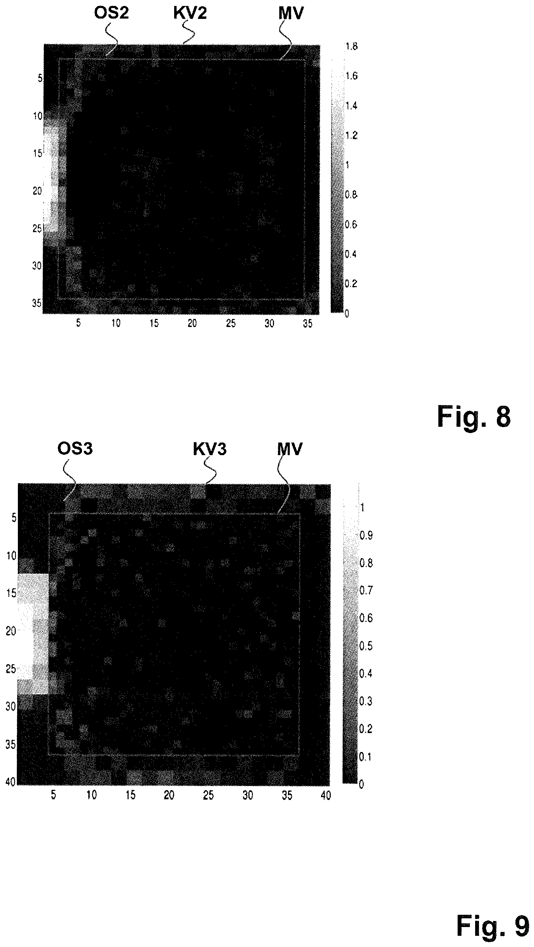

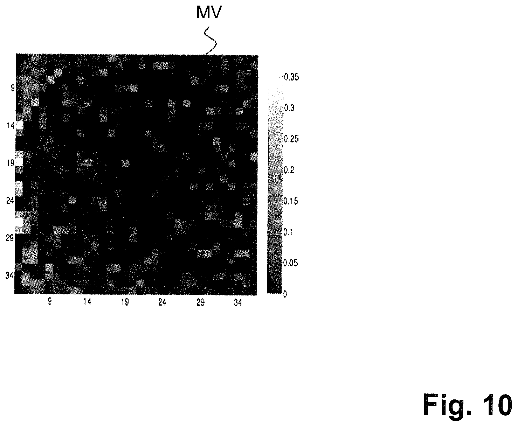

**Fig. 8 und 9** zeigen simulierte rekonstruierte Daten mit einem Overscan von zwei Voxelreihen (Fig. 8) bzw. zwei OS-Voxelreihen und einer OS-Voxelgröße von vier Voxeln (Fig. 9). Die Projektion der externen Magnetpartikelverteilung in das Messvolumen ist in Fig. 8 deutlich reduziert und in Fig. 9 kaum mehr erkennbar. In den Overscan-Bereichen OS2, OS3 ist ein Bereich erkennbar, der eine hohe Signalintensität aufweist, jedoch nicht die wirkliche Magnetpartikelverteilung wiedergibt. Die rekonstruierte Daten für die Overscan-Bereiche OS2, OS3 sind daher als nicht vertrauenswürdig einzustufen. Gemäß dem erfindungsgemäßen Verfahren werden die für den Overscan-Bereich OS2, OS3 rekonstruierte Daten verworfen, so dass sich als Ergebnis der MPI-Messung (MPI-Bild) bspw. die in Fig. 10 gezeigte Magnetpartikelverteilung (statt der in Fig. 9 gezeigten Magnetpartikelverteilung) ergibt.

Mit dem erfindungsgemäßen Verfahren können daher zuverlässige MPI-Daten erhalten werden auch für Messvolumina, die von Bereichen mit hoher Magnetpartikeldichte umgeben sind.

### Referenzliste

[01] T. Knopp, S. Biederer, T. F. Sattel, J. Rahmer, J. Weizenecker, B. Gleich, J. Borgert, T. M. Buzug; "2D model-based reconstruction for magnetic particle imaging"; Medical Physics, 37(2):485-491, 2010
[02] B. Gleich, J. Weizenecker, J. Borgert; "Experimental results on fast 2D-encoded magnetic particle imaging" Physics in Medicine and Biology, 53(6):N81-N84, Mar. 2008
[03] J. Weizenecker, B. Gleich, J. Rahmer, H. Dahnke, J. Borgert; "Threedimensional real-time in vivo magnetic particle imaging"; Physics in Medicine and Biology, 54(5):L1-L10, 2009

### Bezugszeichenliste

- d, d', d": Dicke des Overscan-Bereichs
- KV, KV1, KV2, KV3: Kalibrationsvolumen
- MS: Magnetpartikel-Streifen
- MV: Messvolumen
- OS2, OS3: Overscan-Bereich

## Patentansprüche

1. MPI-Verfahren zur Lokalisierung von Magnetpartikeln innerhalb einer Messprobe, wobei ein ortsabhängiges Magnetfeld angelegt wird, welches eine feldfreie Region aufweist, umfassend folgende Verfahrensschritte:
• Festlegen eines Kalibrationsvolumens (KV1, KV2, KV3) und eines Messvolumens (MV), wobei das Kalibrationsvolumen (KV1, KV2, KV3) größer ist als das Messvolumen (MV) und wobei das Messvolumen (MV) vollständig innerhalb des Kalibrationsvolumens (KV1, KV2, KV3) angeordnet ist;
• Erfassen von Kalibrationssignalen Sⱼ und Erstellung einer Systemmatrix S aus den Kalibrationssignalen Sⱼ;
• Aufnahme eines MPI-Messsignals u wobei durch Anlegen eines magnetischen Ansteuerungsfeldes die feldfreie Region mit Hilfe einer Messsequenz durch das Messvolumen (MV) bewegt wird;
• Rekonstruktion einer ortsabhängigen Magnetpartikelkonzentration c mit Magnetpartikelkonzentrationswerten cᵢ innerhalb des Kalibrationsvolumens (KV1, KV2, KV3) aus dem aufgenommenen MPI-Messsignal und der erstellten Systemmatrix S und Zuordnung der Magnetpartikelkonzentrationswerte cᵢ zu Voxeln im Kalibrationsvolumen (KV1, KV2, KV3);
• Verwerfen derjenigen Magnetpartikelkonzentrationswerte cᵢ, welche Voxeln außerhalb des Messvolumens (MV) zugeordnet wurden,
• Erstellen eines MPI-Bildes, welches ausschließlich Magnetpartikelkonzentrationswerte ci enthält, die den Voxeln eines Voxelgitters innerhalb des Messvolumens zugeordnet wurden.

2. Verfahren nach Anspruch 1, **dadurch gekennzeichnet, dass** das Kalibrationsvolumen (KV1, KV2, KV3) das Messvolumen (MV) umgibt.

3. Verfahren nach Anspruch 2, **dadurch gekennzeichnet, dass** das Kalibrationsvolumen (KV1) das Messvolumen (MV) in jeder Richtung (x, -x, y, - y, z, -z) um jeweils ein Voxel überragt.

4. Verfahren nach einem der vorhergehenden Ansprüche, **dadurch gekennzeichnet, dass** im Bereich außerhalb des Messvolumens die Kalibrationssignale mit einer kleineren Auflösung erfasst werden als innerhalb des Messvolumens

5. Verfahren nach Anspruch 4, **dadurch gekennzeichnet, dass** in einem Bereich außerhalb des Messvolumens weniger Kalibrationssignale aufgenommen werden als Voxelpositionen in diesem Bereich vorhanden sind.

6. Verfahren nach einem der Ansprüche 4 oder 5, **dadurch gekennzeichnet, dass** für die Erfassung der Kalibrationssignale außerhalb des Messvolumens (MV) eine größere Kalibrierprobe verwendet wird als für die die Erfassung der Kalibrationssignale innerhalb des Messvolumens (MV).

7. Verfahren nach einem der Ansprüche 4 bis 6, **dadurch gekennzeichnet, dass** für die Erfassung der Kalibrationssignale außerhalb des Messvolumens (MV) ein anderes Voxelgitter als für die Erfassung der Kalibrationssignale innerhalb des Messvolumens (MV) verwendet wird.

8. Verfahren nach einem der vorhergehenden Ansprüche, **dadurch gekennzeichnet, dass** das Verfahren zum Vermessen von Messproben eingesetzt wird, die zumindest einen zum Messvolumen (MV) benachbarten Bereich aufweisen mit einer Magnetpartikelkonzentration, die größer Null, vorzugsweise größer oder gleich der Magnetpartikelkonzentration im Messvolumen (MV) ist.

9. Verfahren nach einem der vorhergehenden Ansprüche, **dadurch gekennzeichnet, dass** zur Erfassung der Kalibrationssignale eine Kalibrationsmessung durchgeführt wird, wobei für verschiedene Positionierungen der Kalibrierprobe innerhalb des Kalibrationsvolumens (KV1, KV2, KV3) jeweils ein Kalibrationssignal Sⱼ detektiert wird, wobei die feldfreie Region während der Detektion jedes Kalibrationssignals Sⱼ durch Anlegen des magnetischen Ansteuerungsfeldes gemäß der Messsequenz durch das Messvolumen (MV) bewegt wird.

10. Verfahren nach Anspruch 9, **dadurch gekennzeichnet, dass** bei der Kalibrationsmessung zuerst für Probenpositionen außerhalb des Messvolumens (MV) und danach für Probenpositionen innerhalb des Messvolumens Kalibrationssignale gemessen werden.

11. Verfahren nach einem der vorhergehenden Ansprüche, **dadurch gekennzeichnet, dass** das Verfahren für in-vivo-Aufnahmen verwendet wird.

## Claims

1. MPI method for localizing magnetic particles within a test sample, wherein a location-dependent magnetic field is applied which has a field-free region, comprising the following process steps:
- determining a calibration volume (KV1, KV2, KV3) and a measurement volume (MV), wherein the calibration volume (KV1, KV2, KV3) is larger than the measurement volume (MV) and wherein the overall measurement volume (MV) is arranged within the calibration volume (KV1, KV2, KV3);
- detecting calibration signals Sⱼ and creating a system matrix S from the calibration signals Sⱼ;
- recording an MPI measuring signal u, wherein through application of a magnetic control field, the field-free region is moved through the measurement volume (MV) by means of a measuring sequence;
- reconstruction of a location-dependent magnetic particle concentration c with magnetic particle concentration values cᵢ within the calibration volume (KV1, KV2, KV3) from the recorded MPI measurement signal and the created system matrix S and association of the magnetic particle concentration values cᵢ with voxels in the calibration volume (KV1, KV2, KV3);
- discarding those magnetic particle concentration values cᵢ which were associated with voxels outside of the measurement volume (MV),
- generating an MPI image which exclusively contains magnetic particle concentration values ci which were associated with the voxels of a voxel grid within the measurement volume.

2. Method according to claim 1, **characterized in that** the calibration volume (KV1, KV2, KV3) surrounds the measurement volume (MV).

3. Method according to claim 2, **characterized in that** the calibration volume (KV1) projects past the measurement volume (MV) in each direction (x, -x, y, -y, z, -z) by one voxel in each case.

4. Method according to any one of the preceding claims, **characterized in that** the calibration signals are detected with a lower resolution in the area outside of the measurement volume than inside the measurement volume.

5. Method according to claim 4, **characterized in that** fewer calibration signals are recorded in an area outside of the measurement volume than voxel positions present **in that** area.

6. Method according to any one of the claims 4 or 5, **characterized in that** a larger calibration sample is used for detecting the calibration signals outside of the measurement volume (MV) than for detecting the calibration signals inside the measurement volume (MV).

7. Method according to any one of the claims 4 to 6, **characterized in that** a different voxel grid is used for detecting the calibration signals outside of the measurement volume (MV) than for detecting the calibration signals inside the measurement volume (MV).

8. Method according to any one of the preceding claims, **characterized in that** the method is used for measuring test samples having at least one area next to the measurement volume (MV) with a magnetic particle concentration which is larger than zero, preferably larger or equal to the magnetic particle concentration in the measurement volume (MV).

9. Method according to any one of the preceding claims, **characterized in that** a calibration measurement is performed for detecting the calibration signals, wherein one calibration signal Sⱼ is detected in each case for different positionings of the calibration sample within the calibration volume (KV1, KV2, KV3), wherein the field-free region is moved through the measurement volume (MV) during detection of each calibration signal Sⱼ through application of the magnetic control field in accordance with the measuring sequence.

10. Method according to claim 9, **characterized in that** in the calibration measurement calibration signals are first measured for sample positions outside of the measurement volume (MV) and subsequently for sample positions inside the measurement volume.

11. Method according to any one of the preceding claims, **characterized in that** the method is used for in vivo recordings.

## Revendications

1. Procédé de MPI pour localiser des particules magnétiques à l'intérieur d'un échantillon de mesure, selon lequel est appliqué un champ magnétique dépendant de la localisation qui présente une région dépourvue de champ, comprenant les étapes de procédé suivantes :
• définition d'un volume d'étalonnage (KV1, KV2, KV3) et d'un volume de mesure (MV), le volume d'étalonnage (KV1, KV2, KV3) étant plus grand que le volume de mesure (MV) et le volume de mesure (MV) étant disposé entièrement à l'intérieur du volume d'étalonnage (KV1, KV2, KV3) ;
• acquisition de signaux d'étalonnage Sⱼ et création d'une matrice du système S à partir des signaux d'étalonnage Sⱼ ;
• enregistrement d'un signal de mesure de MPI u, la région dépourvue de champ étant déplacée à travers le volume de mesure (MV) à l'aide d'une séquence de mesure par application d'un champ magnétique de commande ;
• reconstitution d'une concentration en particules magnétiques c dépendante de la localisation avec des valeurs de concentration en particules magnétiques cᵢ à l'intérieur du volume d'étalonnage (KV1, KV2, KV3) à partir du signal de mesure de MPI enregistré et de la matrice du système S créée et affectation des valeurs de concentration en particules magnétiques cᵢ à des voxels dans le volume d'étalonnage (KV1, KV2, KV3) ;
• rejet des valeurs de concentration en particules magnétiques cᵢ qui ont été affectées à des voxels à l'extérieur du volume de mesure (MV),
• création d'une image de MPI qui contient exclusivement des valeurs de concentration en particules magnétiques cᵢ qui ont été affectées aux voxels d'une grille de voxels à l'intérieur du volume de mesure.

2. Procédé selon la revendication 1, **caractérisé en ce que** le volume d'étalonnage (KV1, KV2, KV3) entoure le volume de mesure (MV).

3. Procédé selon la revendication 2, **caractérisé en ce que** le volume d'étalonnage (KV1) dépasse le volume de mesure (MV) d'un voxel dans toutes les directions (x, -x, y, -y, z, -z).

4. Procédé selon l'une des revendications précédentes, **caractérisé en ce que** les signaux d'étalonnage sont acquis avec une résolution plus petite dans la zone extérieure au volume de mesure qu'à l'intérieur du volume de mesure.

5. Procédé selon la revendication 4, **caractérisé en ce que** moins de signaux d'étalonnage sont enregistrés dans une zone extérieure au volume de mesure qu'il n'existe de positions de voxels dans cette zone.

6. Procédé selon l'une des revendications 4 ou 5, **caractérisé en ce qu'**un échantillon d'étalonnage plus grand est utilisé pour l'acquisition des signaux d'étalonnage à l'extérieur du volume de mesure (MV) que pour l'acquisition des signaux d'étalonnage à l'intérieur du volume de mesure (MV).

7. Procédé selon l'une des revendications 4 à 6, **caractérisé en ce qu'**une autre grille de voxels est utilisée pour l'acquisition des signaux d'étalonnage à l'extérieur du volume de mesure (MV) que pour l'acquisition des signaux d'étalonnage à l'intérieur du volume de mesure (MV).

8. Procédé selon l'une des revendications précédentes, **caractérisé en ce que** le procédé est utilisé pour mesurer des échantillons de mesure qui présentent au moins une zone adjacente au volume de mesure (MV) avec une concentration en particules magnétiques qui est supérieure à zéro, de préférence supérieure ou égale à la concentration en particules magnétiques dans le volume de mesure (MV).

9. Procédé selon l'une des revendications précédentes, **caractérisé en ce qu'**une mesure d'étalonnage est effectuée pour acquérir les signaux d'étalonnage, un signal d'étalonnage Sⱼ étant chaque fois détecté pour différents positionnements de l'échantillon d'étalonnage à l'intérieur du volume d'étalonnage (KV1, KV2, KV3), la région dépourvue de champ étant, pendant la détection de chaque signal d'étalonnage Sⱼ, déplacée à travers le volume de mesure (MV) selon la séquence de mesure par application du champ magnétique de commande.

10. Procédé selon la revendication 9, **caractérisé en ce que**, lors de la mesure d'étalonnage, des signaux d'étalonnage sont mesurés d'abord pour des positions d'échantillon situées à l'extérieur du volume de mesure (MV) et ensuite pour des positions d'échantillon situées à l'intérieur du volume de mesure.

11. Procédé selon l'une des revendications précédentes, **caractérisé en ce que** le procédé est utilisé pour des enregistrements in vivo.
